# EUROPEAN PATENT APPLICATION

(11) **EP 2 333 536 A2**
(43) Date of publication of application: **15.06.2011**
(21) Application number: 10193064.2
(22) Date of filing: 30.11.2010
(51) Int. Cl.: G01N 29/032, G01N 29/22

(54) **Sensors for detecting gas in, and pressure of, a liquid**

(30) Priority: 30.11.2009 GB 0920928; 20.07.2010 US 839444
(71) Applicant: Morgan Electro Ceramics Limited, Thornhill Southhampton Hampshire SO19 7TG (GB)
(72) Inventor: Miles, Richard, Southampton, Hampshire SO19 7TG (GB); Beswick, Tony, Southampton, Hampshire SO19 7TG (GB); Wield, Graham, Southampton, Hampshire SO19 7TG (GB)
(74) Representative: Phillips & Leigh

(57) **Abstract**

A sensor comprises a body having a channel adapted to receive a hose, the channel comprising opposed fixed walls, a transmitter in one of said walls and a receiver in the opposed wall, and a pressure sensor disposed between the opposed walls.

## Description

This invention relates to sensors for detecting gas in a liquid and pressure of the liquid and particularly relates to sensors for detecting gas bubbles in liquid flowing in a hose, and detecting abnormal pressure in the hose.

Sensors for detecting gas bubbles in liquid flowing in a hose are known, and are commonly used in medical applications, such as medical infusion apparatus, where introduction of gas bubbles into a patient's bloodstream can have unfortunate and occasionally fatal results.

Known sensors use a transmitter and receiver pair situated either side of the hose, the transmitter transmitting a signal which passes through the liquid, and the receiver detecting the signal. The signal detected from passage through liquid containing gas bubbles differs from that detected from passage through bubble-free liquid.

Known sensors use optical or piezoelectric transmitter and receiver pairs, and the present invention is not limited to the use of any particular type of transducer.

One geometry for such a sensor comprises a U-shaped body acting as a saddle in which the hose sits. A closure is provided to restrain the hose in the U-shaped body and the transmitter and receiver pair are mounted one in the U-shaped body at the base of the U, and one within the closure. The closure may also be an opposed U-shaped body with the transmitter or receiver mounted at the base of the U. Where a piezoelectric transmitter and receiver pair is used, the pressure between the U-shaped body and the closure ensures good contact of the transmitter and receiver pair with the hose. However because the transmitter and receiver pair are not at a fixed separation, and with some arrangements may not always face each other square on, this can complicate matters and interfere with the nature of the signal received. Such sensors using a transmitter and receiver pair mounted separately in the base of a U-shaped body and in a closure are shown in US4722224 and US5123275.

In an alternative arrangement, a U-shaped body again acts as a saddle in which the hose sits. However the U-shaped body carries both of the transmitter and receiver pair, which are mounted facing each other in the opposed limbs of the U. A closure may be provided to restrain the hose in the U-shaped body and, where a piezoelectric transmitter and receiver pair is used, to force the hose into close contact with the transmitter and receiver. Alternatively, the resilience of the hose may be employed to ensure adequate contact by squeezing the hose into the limbs of the U. Such sensors using a U-shaped body carrying both of the transmitter and receiver pair are shown in US4418565, US4668945, and US5394732.

It is also useful in some circumstances to be able to detect a change in pressure in a hose. As an example, in the medical field, if a hose used for introducing fluids into a patient is blocked, then the pressure in the hose may increase. Alternatively, if the hose becomes disconnected, or blocked prior to the pump, the pressure may drop. In either case, a signal from a pressure sensor may be used to switch off a pump, or to close a valve, or to trigger an alarm as appropriate. Typically, pressure sensors are placed to one side of a channel and are opposed by a surface providing a reaction. For example DE19646701 shows a laterally extending channel with a force sensor opposed by the roof of the channel.

Such pressure sensors may be placed remote from the transmitter and receiver pair. However US6085574 discloses a sensor in which the transmitter receiver pair are relatively movable, and a detector is used to detect relative displacement between the transmitter and receiver pair and thereby provide an indication of excess pressure in the hose, which deforms under the excess pressure. Such a system suffers the drawbacks that the provision of relative motion between the transmitter receiver pair complicates the engineering of the sensor, and that this relative movement can interfere with the nature of the signal received, since the path length may vary.

The present invention provides an integrated gas bubble detector and pressure sensor in which the transmitter and receiver pair are at a fixed separation.

US2009/0078047 discloses an integrated gas bubble detector and pressure sensor comprising a body having a channel adapted to receive a hose, the channel comprising opposed fixed walls, a transmitter in one of said walls and a receiver in the opposed wall, and having a pressure sensor mounted on one to the walls. This arrangement displaces the pressure sensor from the area where the bubble detection takes place and provides little advantage over separate pressure and bubble detectors. In such an arrangement it may be possible for the pressure sensor to engage with the hose while the hose is displaced from a measuring position for the bubble sensor, or vice versa, so resulting in incorrect operation.

The present invention, by locating the pressure sensor in the base of the channel permits pressure and bubble detection to occur at approximately the same place in the hose, and thereby much reduces the risk of engagement of one sensor without engagement with the other sensor.

Accordingly, the present invention provides, a sensor comprising a body having a channel adapted to receive a hose, the channel comprising opposed fixed walls, a transmitter in one of said walls and a receiver in the opposed wall, and a pressure sensor disposed between the opposed walls.

Further features of the invention will be apparent from the claims and as exemplified in the following non-limitative description and accompanying drawings in which:-
Fig. 1 shows a front sectional view of a sensor in accordance with the invention;
Fig. 2 shows a bottom plan view of the sensor of Fig. 1;
Fig. 3 shows a side elevation of the sensor of Fig. 1; and
Fig. 4 is a schematic view showing an alternative construction in accordance with the invention.
Fig. 5 is an exploded view of a sensor in accordance with the present invention.

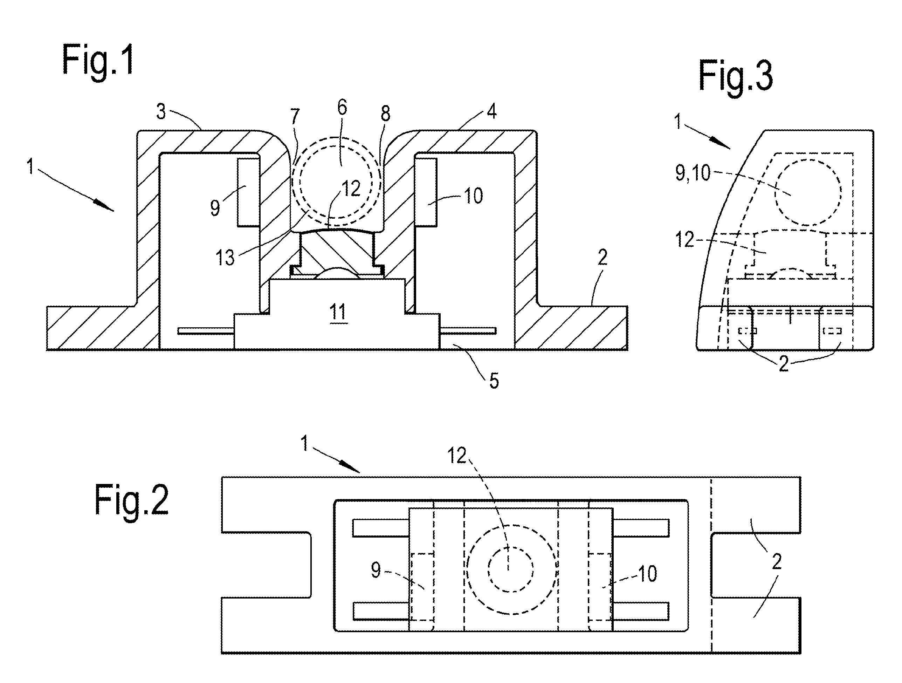

Figs. 1 to 2 show a body 1 having mounting lugs 2 for securing it to a surface.

The body is generally U-shaped having limbs 3,4 and a base 5 defining between them a channel 6 having opposed walls 7,8. The body may be made of any suitable material capable of transmitting ultrasonic vibrations through the walls of the channel. Plastics materials may be suitable, e.g. a moulding from a plastic material from the family PC/ABS (polycarbonate-acrylonitrile butadiene styrene blend) an example being Cycoloy resin CX7240 available from GE Plastics.

The channel 6 may receive a hose of appropriate dimensions in a tight fit.

In limb 3 is mounted a piezoelectric transmitter 9 and in limb 4 is mounted a piezoelectric receiver 10. The transmitter and receiver may conveniently be piezoceramic devices and may be a DOD Navy type II piezo ceramic material [e.g. a lead zirconate titanate material such as PZT-5A obtainable from Morgan Electroceramics Limited] resonating at approx 2MHz or approx 1.5MHz, with a matching layer odd denominations of 1/4 wavelength thick e.g. 1/4, 3/4, 5/4, 7/4. The transmitter / receiver can be of fixed or sweeping frequency.

In the base 5 is mounted a pressure sensor 11. A plunger 12 is mounted for movement in the base and is disposed to contact both against a hose received in channel 6 and the pressure sensor 11. The plunger 12 acts to transmit pressure to the pressure sensor 11. Conveniently the sensor may be a commercial force sensor having a force transmitting plunger that bears on a piezoresistor. Such sensors include the Honeywell FS series of sensors such as the FSS1500 (http://sensing.honeywell.com/index.cfm/ci_id/140822/la_id/1/document/1/re_id/0) or an HFD500 as manufactured by Hokuriku (http://www.hdk.co.jp/pdf/eng/e1381aa.pdf).

The plunger 12 may be shaped so as to be wider at the base than where it protrudes into the channel so as to retain it in place. Alternative retention means may be used, e.g co-operating pins and slots in the plunger and base. As a further alternative, the plunger may be a straight rod that is retained by a moulded plastic guide with a straight bore and held in place by a membrane that acts as a moisture barrier as well.

Alternatively, the force transmitting plunger of a suitable sensor may protrude through an aperture in the base 5 to make direct contact with the hose.

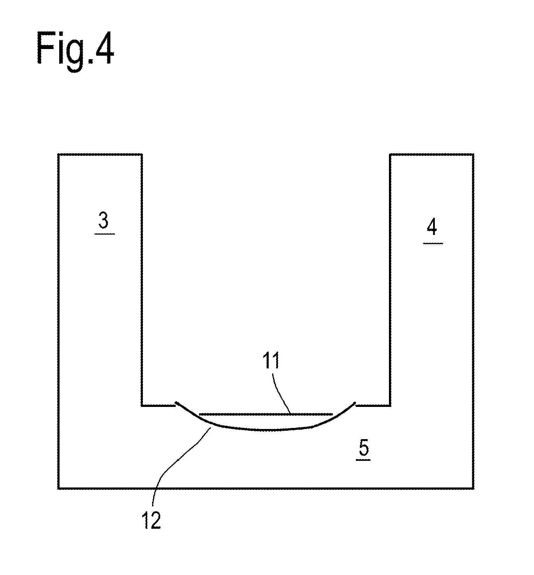

An alternative arrangement is shown schematically in Fig.4 in which the sensor body protrudes into a cut-out portion 12 in the base 5 of the channel.

In operation, a hose 13 carrying liquid is placed in the channel in a tight fit such that good contact is made with the opposed walls 7,8 and the plunger 12. Ultrasonic vibrations from transmitter 9 pass through the wall 7, through the hose, through the fluid carried in the hose, through the wall 8 to the receiver 10. Change in the signal received at 10 is processed to provide an indication of gas in the fluid in known manner.

Pressure sensor 11 gives an indication of the pressure of fluid in hose 13. Both positive and negative pressure changes can be detected, as the action of the tube on the piston acts as a preloaded pressure.

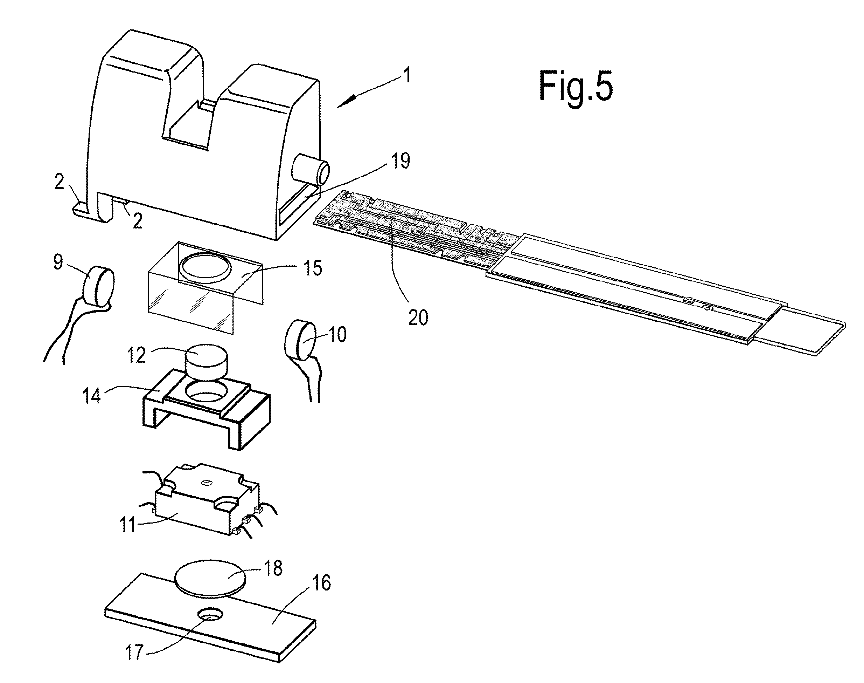

Fig. 5 shows an exploded view of a sensor in accordance with the present invention, in which like parts receive the same reference numbers as in Figs. 1 to 3.

Body 1 houses piezoelectric transmitter and receiver 9,10 and pressure sensor 11. A piston 12 is mounted in a piston guide 14 and retained by polyurethane membrane 15.

A base 16 secures the pressure sensor 11, piston 12, piston guide 14, and polyurethane membrane 15 within the body 1. The base 16 has a breathing hole 17 to permit pressure equalisation and prevent changes in atmospheric pressure being detected as changes in force by the pressure sensor 11. A breathable membrane 18 is provided to permit air entry through the breathing hole 17.

The body 1 has an aperture 19 to receive a flexible printed circuit 20 permitting wiring to the piezoelectric transmitter and receiver 9,10 and the pressure sensor 11.

Typical dimensions of a sensor such as shown in figures 1 to 4 are:-

| | |
|---|---|
| Overall length | 26mm |
| Overall height | 10mm |
| Overall width | 8mm |
| Channel width [distance from wall 7 to wall 8] | 4.25mm |
| Channel depth | 4.5mm |
| Wall thickness [walls 7 and 8] | 1.35mm |

When the pressure sensor is aligned to detect at least in part pressure in the tube between the piezoelectric transmitter and receiver, a compact arrangement is achieved, which furthermore has the advantage that the sensor either detects both pressure and bubbles at the same time, or sense nothing at all. In contrast, if the pressure and piezoelectric transmitter and receiver are separated spatially, e.g. along a slot, so that pressure detection is on a different part of the tube than lies between the piezoelectric transmitter and receiver, it is possible for pressure to be measured while the tube is not properly positioned between the pressure and piezoelectric transmitter and receiver.

In use the sensor can be connected to any suitable apparatus capable of providing a signal to the to the transmitter, capable of receiving the signal from the receiver, capable of detecting variations in the signal from the receiver and to thereby provide an indication of the presence of gas in the liquid, and capable of receiving a signal from the pressure sensor and thereby indicate a change in pressure in the liquid. Of course, the sensor can be used in co-operation with apparatus that does not have all of this functionality if only gas in fluid or pressure detection is required rather than both.

The sensor is particularly useful for use in medical infusion apparatus.

## Claims

1. A sensor comprising:-
• a body (1) having a channel (6) adapted to receive a hose and comprising two opposed fixed walls (7,8) and a channel base (5),
• a transmitter (9) in one of said walls; and
• a receiver (10) in the opposed wall,
**characterised in that** a pressure sensor (11) is mounted in the channel base (5) and disposed between the opposed walls (7,8).

2. A sensor, as claimed in Claim 1, in which the channel (6) is a channel in a generally U-shaped body, limbs (3,4) of the U defining the opposed walls (7,8).

3. A sensor, as claimed in Claim 1 or Claim 2, in which the transmitter (9) and receiver (10) are ultrasonic devices.

4. A sensor, as claimed in Claim 3, in which the ultrasonic devices are piezoelectric devices

5. A sensor, as claimed in Claim 1, in which the pressure sensor comprises a force transmitting plunger (12) that bears on a piezoresistor.

6. A sensor, as claimed in Claim 2, in which a plunger (12) is mounted for movement in the base (5) and is disposed to contact the pressure sensor (11), and in use to contact a hose received in the channel.

7. A sensor, as claimed in Claim 1, in which the body (1) is formed from plastics material.

8. Apparatus for detection of gas in, and sensing pressure of, a liquid in a hose, comprising:-
a sensor (1) as claimed in any one of Claims 1 to 7,
means to provide a signal to the transmitter (9)
means to receive the signal from the receiver (10)
means to detect variations in the signal from the receiver (10) and to thereby provide an indication of the presence of gas in the liquid
means to receive a signal from the pressure sensor (11) and thereby indicate a change in pressure in the liquid.

9. Medical infusion apparatus comprising apparatus as claimed in Claim 8.

10. Methods of infusing substances into human or animal subjects comprising the use of medical infusion apparatus as claimed in Claim 9.
